# FASCICULE DE BREVET EUROPEEN

(11) **EP 3 085 416 B1**
(45) Date de publication et mention de la délivrance du brevet: **26.02.2020**
(21) Numéro de dépôt: 16166518.7
(22) Date de dépôt: 22.04.2016
(51) Int. Cl.: A61Q 11/00, A61K 8/97, A61K 8/02, A61K 36/45, A61K 8/86, A61K 8/92

(54) **COMPOSITION LIQUIDE OU SEMI-LIQUIDE À BASE DE PLANTE DE LA FAMILLE DES ERICACEAE POUR UNE APPLICATION BUCCALE**
FLÜSSIGE ODER HALBFLÜSSIGE ZUSAMMENSETZUNG AUF PFLANZENBASIS DER ERICACEAE-FAMILIE FÜR DIE BUKKALE ANWENDUNG
LIQUID OR SEMI-LIQUID COMPOSITION MADE OF PLANTS FROM THE ERICACEAE FAMILY FOR ORAL APPLICATION

(30) Priorité: 23.04.2015 FR 1553624
(43) Date de publication de la demande: 26.10.2016
(73) Titulaire: Laboratoires Chemineau, 37210 Vouvray (FR)
(72) Inventeur: AGNUS, Benoît, 37110 Villedomer (FR); RENAUD, Marie, 37700 La-Ville-aux-Dames (FR)
(74) Mandataire: Plasseraud IP

(56) Documents cités:
- EP-A1- 1 661 576
- WO-A1-00/71090
- WO-A1-96/28135
- US-A- 5 980 869
- US-A1- 2005 158 381
- US-A1- 2009 156 563
- DATABASE GNPD [Online] MINTEL; juin 2010 (2010-06), "Siberry Strengthening Toothpaste", XP002758736, Database accession no. 1330949
- DATABASE GNPD [Online] MINTEL; juillet 2009 (2009-07), "Cool Mint Mouthwash", XP002758737, Database accession no. 1143426
- KARINE FEGHALI ET AL: "Cranberry Proanthocyanidins: Natural Weapons against Periodontal Diseases", JOURNAL OF AGRICULTURAL AND FOOD CHEMISTRY, vol. 60, no. 23, 13 juin 2012 (2012-06-13) , pages 5728-5735, XP055153608, ISSN: 0021-8561, DOI: 10.1021/jf203304v

## Description

La présente invention a pour objet une composition liquide ou semi-liquide à base de plante de la famille des Ericaceae, à savoir de canneberge, destinée à une application buccale.

La canneberge, encore appelée grande airelle rouge d'Amérique du Nord ou par son nom anglais « cranberry », est une plante vivace qui pousse à l'état sauvage et qui possède des baies rouges. Le jus de canneberge entre dans la composition de nombreux jus et cocktails : son goût acidulé, astringent et âpre le différencie des autres jus et nectars de fruits.

Le fruit de la canneberge (*Vaccinium macrocarpon*) est reconnu pour ses qualités thérapeutiques, en premier lieu en tant qu'antioxydant. La canneberge est en effet riche en vitamine C et en antioxydants, en particulier des flavonoïdes.

Le fruit de la canneberge comprend des proanthocyanidines de type A, composés ayant été décrits comme empêchant certaines bactéries *Escherichia coli* responsables des cystites d'adhérer à la vessie et donc de causer l'infection urinaire. Ne bénéficiant pas de point d'ancrage, ces bactéries sont alors naturellement éliminées par les voies naturelles.

La canneberge est également efficace pour prévenir et/ou traiter la gingivite (inflammation des gencives) et la parodontite (inflammation du parondonte). En effet, la proanthocyanidine, par ses effets antiadhésifs sur certaines bactéries, permet également d'éliminer certaines bactéries dentaires en créant une sorte de film protecteur.

De par ses différentes propriétés, le fruit de la canneberge se révèle donc d'un grand intérêt.

Un des buts de l'invention est ainsi d'élaborer et de mettre au point de nouvelles formulations liquides ou semi-liquides comprenant de la canneberge, pour une application buccale.

Les formulations liquides ou semi-liquides recherchées par les Inventeurs pour la muqueuse buccale sont plus particulièrement des solutions ou dispersions.

Cependant, de par la faible solubilité de la canneberge, l'élaboration de telles compositions est difficile à mettre en œuvre.

Les Inventeurs ont ainsi découvert de manière surprenante que lorsque la canneberge était associée avec un agent solubilisant ou dispersant particulier, à savoir un mélange d'une huile de ricin et d'un éther monobutylique, alors la cannerberge devenait plus soluble et miscible dans les formulations de l'invention.

La présente invention a ainsi pour objet l'utilisation d'un agent solubilisant ou dispersant particulier pour solubiliser un extrait de canneberge dans une composition liquide ou semi-liquide, ladite composition formant plus particulièrement une solution ou dispersion.

On entend par « solution » un mélange homogène résultant de la dissolution d'un ou plusieurs soluté(s) dans un solvant.

La « dispersion » résulte de la fragmentation d'une substance en particules de petite taille distribuée de façon homogène au sein d'une autre substance. Ces deux substances ne peuvent pas se mélanger et peuvent se trouver sous différents états physique, solide, liquide ou gazeux.

A titre d'exemples de dispersion, on pourra notamment citer les émulsions et les suspensions.

Dans la présente invention, par « solubiliser un extrait de canneberge dans une composition liquide ou semi-liquide » on entend à la fois :
- permettre la dissolution ou la dispersion de la canneberge dans le solvant de la composition de l'invention,
- permettre la dissolution ou la dispersion de la canneberge dans la composition de l'invention,
- permettre la miscibilité de la canneberge avec les autres constituants de la composition de l'invention et,
- assurer la stabilité physique de la composition de l'invention au cours du temps, c'est-à-dire assurer qu'aucune modification physique de la composition n'apparait après 6 mois de stockage à la lumière et à la température ambiante.

De façon avantageuse, la composition de l'invention est destinée à une application buccale, notamment sous forme de spray buccal, de bain de bouche ou de dentifrice.

La composition de l'invention peut également comprendre une gomme, et notamment un mélange de gommes tel que la gomme d'acacia et la gomme xanthane.

Elle comprend en outre un agent humectant tel que le xylitol.

La présente invention a pour objet une composition liquide ou semi-liquide comprenant une concentration suffisante en canneberge.

La présente invention a également pour objet une composition liquide ou semi-liquide exempte d'alcool éthylique, isopropylique ou isobutylique.

Selon l'invention, on entend par « composition semi-liquide », une composition à demi liquide, qui se présente par exemple sous forme de crème ou de gel, et qui présente une viscosité allant de 2000 cP à 20 000 cP (centipoises), ladite mesure de la viscosité étant effectuée à l'aide d'un appareil de type Brookfield.

Selon l'invention, on entend par composition liquide ou semi-liquide comprenant une concentration « suffisante » de canneberge, une composition comprenant au moins 0,07% en poids de canneberge par rapport au poids total de la composition.

La présente invention a également pour objet une composition liquide ou semi-liquide pour une application buccale caractérisée en ce qu'elle comprend :
- un extrait de canneberge (Vaccinium macrocarpon), et de préférence un extrait du fruit de canneberge,- un agent solubilisant ou dispersant de l'extrait de canneberge qui est un mélange d'une huile de ricin hydrogénée et d'un éther monobutylique de poly(éthylène glycol-co-propylène glycol), et- un agent humectant choisi dans le groupe comprenant le xylitol, le sorbitol, le panthénol, le glycérol, le pentylène glycol, l'acide hyaluronique, le sodium hyaluronate et leurs mélanges, et est de préférence le xylitol.

Selon le mode de réalisation avantageux de l'invention, l'agent solubilisant ou dispersant est un mélange d'une huile de ricin hydrogénée et d'un éther monobutylique de poly(éthylène glycol-co-propylène glycol).

Selon un autre mode de réalisation avantageux, la composition de l'invention se présente sous la forme d'une solution ou dispersion.

La composition de l'invention est encore caractérisée en ce qu'elle comprend un solvant choisi dans le groupe comprenant l'eau, l'eau purifiée, l'eau osmosée, l'eau PPI, l'eau de mer naturelle ou reconstituée, l'eau permutée et leurs mélanges.

Selon l'invention, l'extrait de plante est un extrait de canneberge, encore dénommée *Vaccinium macrocarpon,* et de préférence un extrait du fruit de canneberge.

L'utilisation d'un agent solubilisant ou dispersant tel que défini ci-dessus permet avantageusement de dissoudre ou disperser l'extrait de plante tel que défini ci-dessus dans le solvant de la composition de l'invention.

Selon un mode de réalisation de l'invention, l'extrait de canneberge *Vaccinium macrocarpon* sera plus particulièrement un extrait sec.

Selon un autre mode de réalisation de l'invention, la composition comprend en outre une gomme, choisie dans le groupe comprenant la gomme d'acacia, la gomme xanthane, la gomme de guar, la gomme arabique, et leurs mélanges, et est de préférence un mélange de gomme d'acacia et de gomme xanthane.

La composition comprend un agent humectant choisi dans le groupe comprenant le xylitol, le sorbitol, le panthénol, le glycérol, le pentylène glycol, l'acide hyaluronique, le sodium hyaluronate et leurs mélanges, et est de préférence le xylitol.

Selon encore un autre mode de réalisation de l'invention, la composition comprend en outre au moins un composé choisi parmi :
- un agent antifongique et/ou antimicrobien choisi dans le groupe comprenant du benzoate de sodium, du sorbate de potassium, de l'éthyl hexyl glycérine, de l'hydroxyde de sodium, du glycéryl caprylate, du butylèneglycol, un extrait de fleurs, de préférence un extrait de fleurs *Eugenia caryophyllus* et/ou un extrait de fleurs *Artemisia capillaris* et leurs mélanges,
- un arôme ou un parfum choisi dans le groupe comprenant menthol, eucalyptol, trans-anéthole, limonène, menthone, carvone, alpha-pinène, acétate de menthyl, estragol, p-cymène, beta-pinène, menthofurane, benzoate de benzyle, camphre, alcool benzylique, citral, eugénol, géraniol, linalool, citronellol et leurs mélanges,
- un agent colorant, de préférence choisi dans le groupe comprenant un colorant permettant l'obtention d'une coloration finale rouge, comme par exemple un colorant azoïque,
- un agent de neutralisation de la mauvaise haleine choisi dans le groupe comprenant du lactate de zinc, du chlorure du zinc et leurs mélanges,
- un agent épaississant choisi dans le groupe comprenant un acrylate, un polysaccharide et leurs mélanges.

Selon encore un autre mode de réalisation particulier, dans la composition telle que définie ci-dessus :
- l'extrait de plante est présent en une quantité en poids allant de 0,07% à 1,5%, de préférence de 0,2% à 1%,
- l'agent solubilisant ou dispersant est présent en une quantité en poids allant de 1% à 55%,
- la gomme, et notamment le mélange de gomme d'acacia et de gomme xanthane, est présent en une quantité en poids allant de 0,01% à 1%, de préférence de 0,04% à 0,06%,
- l'agent humectant, et notamment le xylitol, est présent en une quantité en poids allant de 3% à 30%, de préférence de 6% à 11%,
- l'agent antifongique et/ou antimicrobien est présent une quantité en poids allant de 0,05% à 2%, de préférence de 0,1% à 1%,
- l'arôme ou le parfum est présent une quantité en poids allant de 0,5% à 5%, de préférence de 1% à 1,7%,
- l'agent colorant est présent une quantité en poids allant de 0,001% à 0,01%, de préférence de 0,002% à 0,005%,
- l'agent de neutralisation de la mauvaise haleine est présent une quantité en poids allant de 0,01% à 0,5%, de préférence de 0,1% à 0,2%,
- l'agent épaississant est présent une quantité en poids allant de 0,05 à 5%, de préférence de 0,1% à 2,0%,
- le solvant est ajouté en une quantité qsp 100%,
chacune desdites quantités en poids étant définie par rapport au poids total de la composition.

L' agent solubilisant ou dispersant est un mélange d'une huile de ricin hydrogénée et d'un éther monobutylique de poly(éthylène glycol-co-propylène glycol), avantageusement présent dans la composition de l'invention en une quantité en poids allant de 1% à 9%, de préférence de 3% à 6%, par rapport au poids total de la composition.

Un autre objet de l'invention réside encore dans l'utilisation d'une composition telle que définie ci-dessus en tant que bain de bouche, spray buccal ou dentifrice

Selon un mode de réalisation de l'invention, la composition liquide est utilisée en tant que bain de bouche, spray buccal ou dentifrice.

Selon un autre mode de réalisation, la composition semi-liquide de l'invention sera utilisée en tant que crème ou gel dans la muqueuse buccale (dans ce cas la crème ou le gel sera par exemple un dentifrice).

L'invention a encore pour objet un bain de bouche caractérisé en ce qu'il comprend une composition liquide telle que définie ci-dessus.

Le bain de bouche selon l'invention pourra avantageusement être utilisé en association avec de l'eau, de préférence gazeuse, éventuellement enrichie en sels minéraux.

L'invention concerne encore un spray buccal comprenant une composition telle que définie ci-dessus.

La présente invention a également pour objet une composition liquide ou semi-liquide telle que définie ci-dessus pour une utilisation dans la prévention et/ou le traitement de la plaque dentaire, des caries, de la gingivite, de la parodontite et/ou de l'halitose.

L'invention concerne encore une crème et/ou un gel comprenant une composition telle que définie ci-dessus, de préférence une composition semi-liquide.

Ladite crème ou ledit gel seront avantageusement utilisés dans la muqueuse buccale pour les utilisations telles que définies ci-dessus (prévention et/ou traitement de la plaque dentaire, des caries, de la gingivite, de la parodontite et/ou de l'halitose).

Selon un mode de réalisation avantageux, l'invention concerne l'utilisation d'un agent solubilisant ou dispersant pour solubiliser ou disperser un extrait de canneberge dans une composition liquide ou semi-liquide destinée à une application buccale dans laquelle l'agent solubilisant ou dispersant est un mélange d'une huile de ricin hydrogénée et d'un éther monobutylique de poly(éthylène glycol-co-propylène glycol).

Ce mélange est désigné dans les exemples 1 à 3 ci-après par :
« Poly(éthylene glycol copropylène glycol) monobutyl éther »
« Huile de ricin hydrogénée »

L'extrait du fruit *Vaccinium macrocarpon* utilisé dans les exemples 1 à 3 ci-après est un extrait sec provenant de la société Nexira.

Les exemples suivants illustrent l'invention, ils ne la limitent en aucune façon.

### Exemple 1 : composition liquide de l'invention utilisable en tant que bain de bouche

La composition d'un bain de bouche selon l'invention est donnée dans le **Tableau 1** ci-dessous.

| **Dénomination INCI** | **% (m/m)** |
|---|---|
| Extrait du fruit *Vaccinium macrocarpon* | 0,2 |
| Gomme d'acacia et gomme xanthane | 0,05 |
| Xylitol | 10 |
| Polyéthylène glycol | 20 |
| Poly(éthylene glycol copropylène glycol) monobutyl éther | 3 |
| Huile de ricin hydrogénée | |
| Eau | |
| Extrait de fleurs *Eugenia caryophyllus* | 0.1 |
| Extrait de fleurs *Artemisia capillaris* | |
| Glycéryl caprylate | |
| Butylène glycol | |
| Colorant rouge | 0,005 |
| Menthol | |
| Eucalyptol | |
| Trans-anéthole | |
| Limonène | |
| Menthone | |
| Carvone | |
| Alpha-pinène | |
| Acétate de menthyl | |
| Estragol | |
| p-Cymène | 0,7 |
| Beta-Pinène | |
| Menthofurane | |
| Benzoate de benzyle | |
| Camphre | |
| Alcool benzylique | |
| Citral | |
| Eugénol | |
| Géraniol | |
| Linalool | |
| Citronellol | |
| Eau purifiée | Qsp 100 |

### Exemple 2 : composition liquide de l'invention utilisable en tant que bain de bouche concentré

La composition d'un bain de bouche concentré selon l'invention est donnée dans le **Tableau 2** ci-dessous.

| **Dénomination INCI** | **% (m/m)** |
|---|---|
| Extrait du fruit *Vaccinium macrocarpon* | 0,4 |
| Gomme d'acacia et gomme xanthane | 0,1 |
| Xylitol | 20 |
| Polyéthylène glycol | 40 |
| Poly(éthylene glycol copropylène glycol) monobutyl éther | |
| Huile de ricin hydrogénée | 6 |
| Eau | |
| Extrait de fleurs *Eugenia caryophyllus* | |
| Extrait de fleurs *Artemisia capillaris* | |
| Glycéryl caprylate | 0.1 |
| Butylène glycol | |
| Colorant rouge | 0,01 |
| Menthol | |
| Eucalyptol | |
| Trans-anéthole | |
| Limonène | |
| Menthone | |
| Carvone | |
| Alpha-pinène | |
| Acétate de menthyl | |
| Estragol | |
| p-Cymène | 1,4 |
| Beta-Pinène | |
| Menthofurane | |
| Benzoate de benzyle | |
| Camphre | |
| Alcool benzylique | |
| Citral | |
| Eugénol | |
| Géraniol | |
| Linalool | |
| Citronellol | |
| Eau purifiée | Qsp 100 |

### Exemple 3 : composition liquide de l'invention utilisable en tant que spray buccal

La composition du spray buccal est donnée dans le **Tableau 3** ci-dessous.

| **Dénomination INCI** | **% (m/m)** |
|---|---|
| Extrait du fruit *Vaccinium macrocarpon* | 0,5 |
| Gomme d'acacia et gomme xanthane | 0,05 |
| Xylitol | 10 |
| Polyéthylène glycol | 20 |
| Poly(éthylene glycol copropylène glycol) monobutyl éther | |
| Huile de ricin hydrogénée | 5 |
| Eau | |
| Extrait de fleurs *Eugenia caryophyllus* | |
| Extrait de fleurs *Artemisia capillaris* | |
| Glycéryl caprylate | 0.1 |
| Butylène glycol | |
| Lactate de zinc | 0,1 |
| Menthol | |
| Eucalyptol | |
| Trans-anéthole | |
| Limonène | |
| Menthone | |
| Carvone | |
| Alpha-pinène | |
| Acétate de menthyl | |
| Estragol | |
| p-Cymène | 1,5 |
| Beta-Pinène | |
| Menthofurane | |
| Benzoate de benzyle | |
| Camphre | |
| Alcool benzylique | |
| Citral | |
| Eugénol | |
| Géraniol | |
| Linalool | |
| Citronellol | |
| Eau purifiée | Qsp 100 |

### Exemple 4: stabilité des compositions de l'invention

Des essais de stabilité des compositions décrites dans les tableaux 1 à 3 ci-dessus ont été conduits.

Toutes les compositions se sont avérées stables bactériologiquement et pharmacocinétiquement à température ambiante allant de 15 à 20°C.

## Revendications

1. Composition liquide ou semi-liquide pour une application buccale **caractérisée en ce qu'**elle comprend :
- un extrait de canneberge (*Vaccinium macrocarpon*), et de préférence un extrait du fruit de canneberge,
- un agent solubilisant ou dispersant de l'extrait de canneberge qui est un mélange d'une huile de ricin hydrogénée et d'un éther monobutylique de poly(éthylène glycol-co-propylène glycol), et
- un agent humectant choisi dans le groupe comprenant le xylitol, le sorbitol, le panthénol, le glycérol, le pentylène glycol, l'acide hyaluronique, le sodium hyaluronate et leurs mélanges, et est de préférence le xylitol.

2. Composition selon la revendication 1, **caractérisée en ce qu'**elle se présente sous la forme d'une solution ou d'une dispersion.

3. Composition selon la revendication 1 ou la revendication 2, **caractérisée en ce qu'**elle comprend en outre un solvant choisi dans le groupe comprenant l'eau, l'eau purifiée, l'eau osmosée, l'eau PPI, l'eau de mer naturelle ou reconstituée, l'eau permutée et leurs mélanges.

4. Composition selon l'une quelconque des revendications 1 à 3, **caractérisée en ce qu'**elle comprend en outre une gomme choisie dans le groupe comprenant la gomme d'acacia, la gomme xanthane, la gomme de guar, la gomme arabique et leurs mélanges, et est de préférence un mélange de gomme d'acacia et de gomme xanthane.

5. Composition selon l'une quelconque des revendications 1 à 4, **caractérisée en ce qu'**elle comprend en outre au moins un composé choisi parmi :
- un agent antifongique et/ou antimicrobien choisi dans le groupe comprenant du benzoate de sodium, du sorbate de potassium, de l'éthyl hexyl glycérine, de l'hydroxyde de sodium, du glycéryl caprylate, du butylèneglycol, un extrait de fleurs, de préférence un extrait de fleurs *Eugenia caryophyllus* et/ou un extrait de fleurs *Artemisia capillaris* et leurs mélanges,
- un arôme ou un parfum choisi dans le groupe comprenant menthol, eucalyptol, trans-anéthole, limonène, menthone, carvone, alpha-pinène, acétate de menthyl, estragol, p-cymène, beta-pinène, menthofurane, benzoate de benzyle, camphre, alcool benzylique, citral, eugénol, géraniol, linalool, citronellol, et leurs mélanges,
- un agent colorant, de préférence choisi dans le groupe comprenant un colorant permettant l'obtention d'une coloration finale rouge, comme par exemple un colorant azoïque,
- un agent de neutralisation de la mauvaise haleine choisi dans le groupe comprenant du lactate de zinc, du chlorure du zinc et leurs mélanges,
- un agent épaississant choisi dans le groupe comprenant un acrylate, un polysaccharide et leurs mélanges.

6. Composition selon l'une quelconque des revendications 1 à 5, **caractérisée en ce que** :
- l'extrait de canneberge est présent en une quantité en poids allant de 0,07% à 1,5%, de préférence de 0,2% à 1%,
- l'agent solubilisant ou dispersant est présent en une quantité en poids allant de 1% à 9%, de préférence de 3% à 6%,
- l'agent humectant, et notamment le xylitol, est présent en une quantité en poids allant de 3% à 30%, de préférence de 6% à 11%,
- la gomme, et notamment le mélange de gomme d'acacia et de gomme xanthane, est présent en une quantité en poids allant de 0,01% à 1%, de préférence de 0,04% à 0,06%,
- l'agent antifongique et/ou antimicrobien est présent une quantité en poids allant de 0,05% à 2%, de préférence de 0,1% à 1,
- l'arôme ou le parfum est présent une quantité en poids allant de 0,5% à 5%, de préférence de 1% à 1,7%,
- l'agent colorant est présent une quantité en poids allant de 0,001% à 0,01%, de préférence de 0,002% à 0,005%,
- l'agent de neutralisation de la mauvaise haleine est présent une quantité en poids allant de 0,01% à 0,5%, de préférence de 0,1% à 0,2%,
- l'agent épaississant est présent une quantité en poids allant de 0,05 à 5%, de préférence de 0,1% à 2,0%,
- le solvant est ajouté en une quantité qsp 100%,
chacune desdites quantités en poids étant définie par rapport au poids total de la composition.

7. Bain de bouche **caractérisé en ce qu'**il comprend une composition liquide selon l'une quelconque des revendications 1 à 6.

8. Utilisation d'un bain de bouche selon la revendication 7, en association avec de l'eau, de préférence gazeuse, éventuellement enrichie en sels minéraux.

9. Spray buccal **caractérisé en ce qu'**il comprend une composition selon l'une quelconque des revendications 1 à 6.

## Patentansprüche

1. Flüssige oder halbflüssige Zusammensetzung zur oralen Anwendung, **dadurch gekennzeichnet, dass** sie umfasst:
- einen Preiselbeerextrakt (*Vaccinium macrocarpon*), und bevorzugt einen Extrakt aus der Preiselbeerfrucht,
- ein Lösungs- oder Dispersionsmittel des Preiselbeerextrakts, das eine Mischung aus einem hydrierten Rizinusöl und einem Poly(ethylenglykol-copropylenglykol)monobutylether ist, und
- ein Befeuchtungsmittel, gewählt aus der Gruppe umfassend Xylitol, Sorbitol, Panthenol, Glycerin, Pentylenglykol, Hyaluronsäure, Natriumhyaluronat und Mischungen daraus, und welches bevorzugt Xylitol ist.

2. Zusammensetzung nach Anspruch 1, **dadurch gekennzeichnet, dass** sie in Form einer Lösung oder Dispersion vorliegt.

3. Zusammensetzung nach Anspruch 1 oder Anspruch 2, **dadurch gekennzeichnet, dass** sie ferner ein Lösungsmittel umfasst, gewählt aus der Gruppe umfassend Wasser, gereinigtes Wasser, Osmosewasser, PPI-Wasser, natürliches oder rekonstituiertes Meerwasser, permutiertes Wasser und Mischungen daraus.

4. Zusammensetzung nach einem der Ansprüche 1 bis 3, **dadurch gekennzeichnet, dass** sie ferner ein Gummi umfasst, gewählt aus der Gruppe umfassend Akaziengummi, Xanthangummi, Guargummi, arabisches Gummi und Mischungen daraus, und bevorzugt eine Mischung aus Akaziengummi und Xanthangummi ist.

5. Zusammensetzung nach einem der Ansprüche 1 bis 4, **dadurch gekennzeichnet, dass** sie ferner mindestens eine Verbindung umfasst, gewählt aus :
- einem antimykotischen und/oder antimikrobiellen Mittel, gewählt aus der Gruppe umfassend Natriumbenzoat, Kaliumsorbat, Ethylhexylglycerin, Natriumhydroxid, Glycerylcaprylat, Butylenglykol, Blütenextrakt, bevorzugt Extrakt aus *Eugenia caryophyllus*-Blüten und/oder Extrakt aus *Artemisia capillaris*-Blüten und Mischungen daraus,
- einem Aroma oder einem Parfum, gewählt aus der Gruppe umfassend Menthol, Eukalyptol, Trans-Anethol, Limonen, Menthon, Carvon, Alpha-Pinen, Menthylacetat, Estragol, p-Cymol, Beta-Pinen, Menthofuran, Benzylbenzoat, Kamfer, Benzylalkohol, Citral, Eugenol, Geraniol, Linalool, Citronellol, und Mischungen daraus,
- ein Färbemittel, bevorzugt gewählt aus der Gruppe umfassend ein Färbemittel zum Erhalten einer roten Endfärbung, wie zum Beispiel einen Azofarbstoff,
- ein Mittel zur Neutralisierung von Mundgeruch, gewählt aus der Gruppe umfassend Zinklactat, Zinkchlorid und Mischungen daraus,
- ein Dickungsmittel, gewählt aus der Gruppe umfasend ein Acrylat, ein Polysaccharid und Mischungen daraus.

6. Zusammensetzung nach einem der Ansprüche 1 bis 5, **dadurch gekennzeichnet, dass**:
- der Preiselbeerextrakt in einer Gewichtsmenge im Bereich von 0,07% bis 1,5%, bevorzugt von 0,2% bis 1% vorliegt,
- das Lösungs- oder Dispersionsmittel in einer Gewichtsmenge im Bereich von 1% bis 9%, bevorzugt von 3% bis 6% vorliegt,
- das Befeuchtungsmittel, und insbesondere Xylitol, in einer Gewichtsmenge im Bereich von 3% bis 30%, bevorzugt 6 % bis 11 % vorliegt,
- das Gummi, und insbesondere die Mischung aus Akaziengummi und Xanthangummi, in einer Gewichtsmenge im Bereich von 0,01 % bis 1 %, bevorzugt 0,04 % bis 0,06 % vorliegt,
- das antimykotische und/oder antimikrobielle Mittel in einer Gewichtsmenge im Bereich von 0,05 % bis 2 %, bevorzugt von 0,1 % bis 1, vorliegt
- der Geschmack oder das Parfum in einer Gewichtsmenge im Bereich von 0,5 % bis 5 %, bevorzugt von 1 % bis 1,7 %, vorliegt,
- das Färbemittel in einer Gewichtsmenge im Bereich von 0,001 % bis 0,01 %, bevorzugt von 0,002 % bis 0,005 %, vorliegt,
- das Neutralisationsmittel für Mundgeruch in einer Gewichtsmenge im Bereich von 0,01 % bis 0,5 %, bevorzugt von 0,1% bis 0,2%, vorliegt,
- das Dickungsmittel in einer Gewichtsmenge im Bereich von 0,05 bis 5%, bevorzugt von 0,1% bis 2,0%, vorliegt,
- das Lösungsmittel in einer Menge qsp 100% zugegeben wird, wobei jede der Gewichtsmengen bezogen auf das Gesamtgewicht der Zusammensetzung definiert ist.

7. Mundwasser, **dadurch gekennzeichnet, dass** es eine flüssige Zusammensetzung nach einem der Ansprüche 1 bis 6 umfasst.

8. Verwendung eines Mundwassers nach Anspruch 7 in Kombination mit Wasser, bevorzugt Sprudelwasser, gegebenenfalls angereichert mit Mineralsalzen.

9. Orales Spray, **dadurch gekennzeichnet, dass** es eine Zusammensetzung nach einem der Ansprüche 1 bis 6 umfasst.

## Claims

1. Liquid or semi-liquid composition for oral application **characterised in that** it comprises:
- an extract of cranberry (*Vaccinium macrocarpon*), and preferably a cranberry fruit extract,
- a solubilising or dispersing agent of the cranberry extract which is a mixture of hydrogenated castor oil and a poly(ethylene glycol-co-propylene glycol) monobutyl ether, and
- a moistening agent chosen in the group comprising xylitol, sorbitol, panthenol, glycerol, pentylene glycol, hyaluronic acid, sodium hyaluronate and mixtures thereof, and is preferably xylitol.

2. Composition according to claim 1, **characterised in that** it is presented in the form of a solution or a dispersion.

3. Composition according to claim 1 or claim 2, **characterised in that** it further comprises a solvent chosen in the group comprising water, purified water, osmosed water, water for injection, natural or reconstituted seawater, permuted water and mixtures thereof.

4. Composition according to any one of claims 1 to 3, **characterised in that** it further comprises a gum chosen in the group comprising acacia gum, xanthan gum, guar gum, gum arabic and mixtures thereof, and is preferably a mixture of acacia gum and xanthan gum.

5. Composition according to any one of claims 1 to 4, **characterised in that** it further comprises at least one compound chosen among:
- an antifungal and/or antimicrobial agent chosen in the group comprising sodium benzoate, potassium sorbate, ethylhexylglycerin, sodium hydroxide, glyceryl caprylate, butylene glycol, a flower extract, preferably a *Eugenia caryophyllus* flower extract and/or an *Artemisia capillaris* flower extract and mixtures thereof,
- an aroma or a fragrance chosen in the group comprising menthol, eucalyptol, trans-anethole, limonene, menthone, carvone, alpha-pinene, menthyl acetate, estragole, p-cymene, beta-pinene, menthofuran, benzyl benzoate, camphor, benzyl alcohol, citral, eugenol, geraniol, linalool, citronellol, and mixtures thereof
- a colouring agent, preferably chosen in the group comprising a dyestuff suitable for obtaining a red final colour, such as for example an azo dye,
- an agent for neutralising bad breath chosen in the group comprising zinc lactate, zinc chloride and mixtures thereof,
- a thickening agent chosen in the group comprising an acrylate, a polysaccharide and mixtures thereof.

6. Composition according to any one of claims 1 to 5, **characterised in that**:
- the cranberry extract is present at a quantity by weight ranging from 0.07% to 1.5%, preferably from 0.2% to 1%,
- the solubilising or dispersing agent is present at a quantity by weight ranging from 1% to 9%, preferably from 3% to 6%,
- the moistening agent, and particularly xylitol, is present at a quantity by weight ranging from 3% to 30%, preferably from 6% to 11%,
- the gum, and particularly the mixture of acacia gum and xanthan gum, is present at a quantity by weight ranging from 0.01% to 1%, preferably from 0.04% to 0.06%,
- the antifungal and/or antimicrobial agent is present at a quantity by weight ranging from 0.05% to 2%, preferably from 0.1% to 1,
- the aroma or flavour is present at a quantity by weight ranging from 0.5% to 5%, preferably from 1% to 1.7%,
- the colouring agent is present at a quantity by weight ranging from 0.001% to 0.01%, preferably from 0.002% to .0.005%,
- the agent for neutralising bad breath is present at a quantity by weight ranging from 0.01% to 0.5%, preferably from 0.1% to 0.2%,
- the thickening agent is present at a quantity by weight ranging from 0.05 to 5%, preferably from 0.1% to 2.0%,
- the solvent is added at a quantity up to 100%,
each of said quantities by weight being defined with respect to the total weight of the composition.

7. Mouthwash **characterised in that** it comprises a liquid composition according to any one of claims 1 to 6.

8. Use of a mouthwash according to claim 7, in associated with water, preferably sparkling water, optionally enriched with mineral salts.

9. Mouth spray **characterised in that** it comprises a composition according to any one of claims 1 to 6.
